# EUROPEAN PATENT APPLICATION

(11) **EP 4 190 337 A1**
(43) Date of publication of application: **07.06.2023**
(21) Application number: 21849087.8
(22) Date of filing: 30.07.2021
(51) Int. Cl.: A61K 35/28, A61P 25/00

(54) **THERAPEUTIC AGENT FOR NERVE DISFUNCTION**

(30) Priority: 31.07.2020 JP 2020130911
(71) Applicant: Neurotech Medical Co., Ltd., Toyonaka-shi, Osaka, 561-0882 (JP)
(72) Inventor: KIHOIN Nagatoshi, Toyonaka-shi, Osaka 561-0882 (JP); OKADA Kunihiko, Toyonaka-shi, Osaka 561-0882 (JP)
(74) Representative: Maiwald GmbH
(86) International application number: PCT/JP2021/028420
(87) International publication number: WO 2022/025276

(57) **Abstract**

The present invention addresses the problem of providing a therapeutic agent containing mesenchymal stem cells, which is more effective for the treatment of a nerve damage. The present invention provides a therapeutic agent for a nerve damage. The therapeutic agent is a preparation for intravenous administration which contains mesenchymal stem cells and/or cells capable of being differentiated into mesenchymal stem cells, and is used in combination with the application of a stimulus to nerves in a patient, in which the application of a stimulus is carried out during a time period between a time point at which the administration of the therapeutic agent is not carried out yet and a time point at which 6 hours has passed after the administration of the therapeutic agent.

## Description

### TECHNICAL FIELD

The present invention relates to a therapeutic agent for treatment of nerve damage.

### BACKGROUND ART

Mesenchymal stem cells are used as therapeutic agents for various diseases. For example, Patent Documents 1 and 2 propose using mesenchymal stem cells to treat nerve diseases.

Patent Documents 1 and 2 also disclose that when administered in combination with rehabilitation, mesenchymal stem cells can produce an enhanced therapeutic effect.
Patent Document 1: PCT International Publication No. WO2017/188457
Patent Document 2: Japanese Unexamined Patent Application (Translation of PCT Application), Publication No. 2013-508013

### DISCLOSURE OF THE INVENTION

### Problems to be Solved by the Invention

However, a further need exists to improve the efficacy of the administration of mesenchymal stem cells for the treatment of nerve damage.

The present invention has been made in light of the circumstances mentioned above. It is an object of the present invention to provide a mesenchymal stem cell-containing therapeutic agent more effective in treating nerve damage. Means for Solving the Problems

The inventors have completed the present invention based on findings that the problem can be solved by combining intravenous administration of mesenchymal stem cells and/or cells capable of differentiating into mesenchymal stem cells with stimulation of the patient's nerve and by adjusting the timing of the stimulation or the area to which the stimulation is applied. More specifically, the present invention provides the following aspects.
(1) A therapeutic agent for treatment of nerve damage, the therapeutic agent being an intravenously administered formulation including mesenchymal stem cells and/or cells capable of differentiating into mesenchymal stem cells, the therapeutic agent being to be administered to a patient in combination with stimulation of a nerve of the patient, the stimulation being to be performed during a period from before to 6 hours after the administration of the therapeutic agent.
(2) A therapeutic agent for treatment of nerve damage, the therapeutic agent being an intravenously administered formulation including mesenchymal stem cells and/or cells capable of differentiating into mesenchymal stem cells, the therapeutic agent being to be administered to a patient in combination with stimulation of a nerve of the patient, the stimulation being to be preferentially applied to at least one selected from the group consisting of an area of nerve damage, an area near an area of nerve damage, and an area capable of compensating for the function of an area of nerve damage.
(3) The therapeutic agent according to aspect (1) or (2), wherein the stimulation is at least one selected from the group consisting of motor stimulation, sensory stimulation, electrical stimulation, magnetic stimulation, language stimulation, and higher brain function stimulation.

### Effects of the Invention

The present invention provides a mesenchymal stem cell-containing therapeutic agent more effective in treating nerve damage.

### PREFERRED MODE FOR CARRYING OUT THE INVENTION

Hereinafter, embodiments of the present invention will be described, which are not intended to limit the present invention.

### <Therapeutic Agent>

The therapeutic agent of the present invention for nerve damage (hereinafter also referred to as "the therapeutic agent of the present invention") is an intravenously administered formulation including mesenchymal stem cells and/or cells capable of differentiating into mesenchymal stem cells. The therapeutic agent of the present invention is to be administered to a patient in combination with stimulation of a nerve of the patient, and the modes of use of the therapeutic agent of the present invention include the two modes defined below. The therapeutic agent of the present invention may have either or both of the following two modes of use.
(1) A mode in which the stimulation is applied during a period from before to 6 hours after the administration of the therapeutic agent.
(2) A mode in which the stimulation is preferentially applied to at least one selected from the group consisting of an area of nerve damage, an area near an area of nerve damage, and an area capable of compensating for the function of an area of nerve damage.

It is previously known that intravenous administration of mesenchymal stem cells can be useful for treating nerve damage. Patent Documents 1 and 2 also disclose that the intravenous administration of mesenchymal stem cells combined with rehabilitation (full-body workout) provides an enhanced therapeutic effect. However, none of Patent Documents 1 and 2 disclose any relationship between the timing of the intravenous administration of mesenchymal stem cells and the timing of rehabilitation. In this regard, the inventors have found that the therapeutic effect of administration of mesenchymal stem cells is enhanced by patient's nerve stimulation, in particular, performed during a period from before to 6 hours after the administration. The inventors have also found that the therapeutic effect of intravenous administration of mesenchymal stem cells is significantly higher when the administration is combined with patient's nerve stimulation than when the administration is combined with full-body workout (e.g., exercise with treadmill or the like).

Moreover, the inventors have unexpectedly found that the therapeutic effect on patients is particularly high when the stimulation is preferentially applied to one or more selected from the group consisting of an area of nerve damage, an area near an area of nerve damage, and an area capable of compensating for the function of an area of nerve damage in patients.

The enhanced efficacy of the nerve damage therapy according to the present invention may be because the adjustment of the timing of the stimulation and/or the adjustment of the area to which the stimulation is applied facilitates the migration of a large number of mesenchymal stem cells to the affected area (e.g., the area of nerve damage) and/or the migration of a large number of cells capable of differentiating into mesenchymal stem cells to the affected area (e.g., the area of nerve damage) to enhance the efficacy of the mesenchymal stem cells and/or the efficacy of the cells capable of differentiating into mesenchymal stem cells.

As used herein, the term "nerve damage" means any type of damage to the nerve itself or to the function of the nerve. In this regard, the cause of nerve damage, the area of nerve damage, or other features should not be limited.

The cause of nerve damage may be any disease. Examples of the causative disease include cerebrovascular diseases, brain tumors, encephalitis, dementia, neurodegenerative diseases, spinal cord damage, myelitis, herniated intervertebral disk, and other central or peripheral nerve diseases or disorders.

As used herein, the term "treating nerve damage" means alleviating or completely curing various symptoms associated with nerve damage (e.g., motor disorders, dysarthria, dysphagia, higher brain dysfunction, dementia, aphasia, parkinsonian syndrome, ataxia, sensory disorders, pain, feeling of cold, numbness, hot flashes). Whether treating nerve damage is effective may be evaluated on the basis of known standers or methods, such as NIHSS (National Institutes of Health Stroke Scale), mRS (modified Rankin scale), AIS (ASIA Impairment Scale), Frankel classification, SIAS (Stroke Impairment Assessment), BRS (Brunnstrom stage), FMA (Fugl Meyer Assessment), MMT (manual muscle testing), FMA (Fugl Meyer Assessment), standard language test of aphasia (SLTA), WAB aphasia test, token test, and MMSE (Mini-Mental State Examination).

As used herein, the term "patient" means any living organism with nerve damage. Examples include mammals, such as humans, monkeys, cows, horses, pigs, dogs, and cats, birds or reptiles, and any other pet animals.

Hereinafter, the features of the therapeutic agent of the present invention will be described in detail.

### (Mesenchymal Stem Cells and Cells Capable of Differentiating into Mesenchymal Stem Cells)

The therapeutic agent of the present invention includes mesenchymal stem cells and/or cells capable of differentiating into mesenchymal stem cells. The therapeutic agent of the present invention may include both mesenchymal stem cells and cells capable of differentiating into mesenchymal stem cells or either mesenchymal stem cells or cells capable of differentiating into mesenchymal stem cells. In the therapeutic agent of the present invention including both mesenchymal stem cells and cells capable of differentiating into mesenchymal stem cells, the ratio between the numbers of the two types of cells may be any suitable value, which is appropriately adjusted depending on the desired therapeutic effect or other factors.

The mesenchymal stem cells (MSCs) used in the present invention may be prepared using any tissues and any method that can provide mesenchyme-derived somatic stem cells with the ability to replicate themselves and to differentiate.

As used herein, the term "cells capable of differentiating into mesenchymal stem cells" means cells capable of normally growing and dividing into mesenchymal stem cells. Such cells have both self-renewal ability and differentiation versatility that allows them to differentiate into a variety of cells. Examples of cells capable of differentiating into mesenchymal stem cells include iPS cells (induced pluripotent stem cells) and ES cells (embryonic stem cells) .

The mesenchymal stem cells and the cells capable of differentiating into mesenchymal stem cells may be isolated from bone marrow, adipose tissues, dental pulp, blood (e.g., peripheral blood, cord blood), placenta, umbilical cord, or other somatic tissues.

The mesenchymal stem cells and the cells capable of differentiating into mesenchymal stem cells may be derived from cells of the patient to whom they are to be administered (autologous cells) or from cells other than those of the patient (xenogeneic cells).

The mesenchymal stem cells may be cells differentiated from ES cells or induced pluripotent stem cells (e.g., iPS cells) or may be lineage cells or Muse cells (multi-lineage differentiating stress enduring cells).

In general, the mesenchymal stem cells to be used remain in an undifferentiated state, which is negative for differentiation markers (e.g., CD24).

The mesenchymal stem cells may be such that the expression of various markers satisfies one of the following conditions:
· The mesenchymal stem cells are positive for at least one selected from CD73, CD90, CD105, or CD200; and
· The mesenchymal stem cells are negative for at least one selected from CD19, CD34, CD45, CD74, CD79α, or HLA-DR.

The mesenchymal stem cells are preferably positive for at least two selected from CD73, CD90, CD105, and CD200 and negative for at least four selected from CD19, CD34, CD45, CD74, CD79α, and HLA-DR, and more preferably positive for CD73, CD90, CD105, and CD200 and negative for CD19, CD34, CD45, CD74, CD79α, and HLA-DR.

The mesenchymal stem cells may be those that have been reported to be useful for the treatment of various diseases, such as those disclosed in PCT International Publication Nos. WO2017/188457 and WO2009/002503 and Japanese Unexamined Patent Application (Translation of PCT Application), Publication No. 2013-508013.

The mesenchymal stem cells may be prepared using any suitable method. The mesenchymal stem cells are preferably prepared by the method disclosed in Japanese Patent No. 4061487. This method includes: adding fresh bone marrow cells onto a culture dish; allowing them to adhere to the culture dish and to grow; and allowing some of the resulting cells to grow again on a culture dish.

The dosage and frequency of administration of the mesenchymal stem cells and/or the cells capable of differentiating into mesenchymal stem cells from the therapeutic agent of the present invention may be appropriately adjusted depending on the desired effect and the conditions (e.g., age, weight, degree of symptom) of the patient to be administered with the therapeutic agent. For example, the number of mesenchymal stem cells and/or cells capable of differentiating into mesenchymal stem cells administered per dose is preferably adjusted to 1 × 10⁴ to 1 × 10⁹, 1 × 10⁵ to 5 × 10⁸, 1 × 10⁶ to 2.5 × 10⁸, or 1 × 10⁷ to 1.5 × 10⁸. The cell concentration of the liquid agent may be adjusted to any suitable level, such as 1 × 10⁴ to 1 × 10⁷ cells/mL.

The time required to administer one dose of the therapeutic agent of the present invention may be adjusted depending on the number of the mesenchymal stem cells to be administered and/or the cells to be administered, capable of differentiating into mesenchymal stem cells, or other conditions. For example, one dose of the therapeutic agent of the present invention may be administered over a period of several minutes to several hours (e.g., 5 minutes to 2 hours).

### (Form of the Therapeutic Agent)

The therapeutic agent of the present invention is an intravenously administered formulation. Therefore, if necessary, the therapeutic agent of the present invention may contain, in addition to the mesenchymal stem cells and/or the cells capable of differentiating into mesenchymal stem cells, a medium (e.g., a physiological buffer solution, sterile water, a saline solution, a glucose solution, a culture medium) and an ingredient known to be used in intravenously administered formulations (e.g., an emulsifier, a surfactant, a stabilizing agent).

The therapeutic agent of the present invention is usually administered by injection or infusion.

### (Stimulation)

The therapeutic agent of the present invention is administered to a patient in combination with stimulation of a nerve of the patient. The stimulation satisfies one or both of the following two requirements. Requirement 1: The stimulation is performed during a period from before to 6 hours after the administration of the therapeutic agent. Requirement 2: The stimulation is preferentially applied to one or more selected from the group consisting of an area of nerve damage, an area near an area of nerve damage, and an area capable of compensating for the function of an area of nerve damage.

The blood concentration of mesenchymal stem cells and/or cells capable of differentiating into mesenchymal stem cells usually increases immediately after the intravenous administration of them but decreases to an undetectable level in the affected area (e.g., the brain) within 72 hours after the administration. If the blood flow to the affected area is increased during the period when the administered mesenchymal stem cells and/or the administered cells capable of differentiating into mesenchymal stem cells remain in or around the affected area (e.g., the area of nerve damage, such as the brain), a correspondingly increased number of mesenchymal stem cells and/or cells capable of differentiating into mesenchymal stem cells will migrate to the affected area to provide an efficiently increased therapeutic effect. Specific methods for that correspond to requirements 1 and 2 above.

In a case where the the therapeutic agent of the present invention is used in a manner that satisfies requirement 1 above, the stimulation (e.g., full-body workout, localized exercise, or motor stimulation, sensory stimulation, electrical stimulation, magnetic stimulation, language stimulation, or higher brain function stimulation, which will be described later) is applied to the patient during a period from before to 6 hours after the administration of the therapeutic agent. The application of the stimulation in such a way as to satisfy requirement 1 above will increase the blood flow to the affected area (e.g., the brain) at a timing when a large amount of mesenchymal stem cells and/or cells capable of differentiating into mesenchymal stem cells remain in the blood, and thus increase the number of mesenchymal stem cells migrating to the affected area and/or the number of cells capable of differentiating into mesenchymal stem cells, migrating to the affected area.

As for requirement 1 above, the expression "from before the administration of the therapeutic agent" means that the time point at which the stimulation is started is before the time point at which the administration of the therapeutic agent is started. As for requirement 1 above, the expression "to 6 hours after the administration of the therapeutic agent" means that the time point at which the stimulation is started is before the time point at which 6 hours have elapsed from the time point at which the administration of the therapeutic agent is started.

In a case where the therapeutic agent of the present invention is used in a manner that satisfies requirement 2 above, the stimulation (e.g., motor stimulation, sensory stimulation, electrical stimulation, magnetic stimulation, language stimulation, or higher brain function stimulation, which will be described later) is applied to the patient at any timing (preferably at a timing that satisfies requirement 1). The application of the stimulation in such a way as to satisfy requirement 2 above will increase the blood flow more preferentially to the affected area (e.g., the area of nerve damage) than to other neural regions and thus increase the number of mesenchymal stem cells migrating to the affected area and/or the number of cells capable of differentiating into mesenchymal stem cells, migrating to the affected area.

As used herein, the term "stimulation" means any type of stimulation that causes a physiological change (e.g., at least one, preferably two or more selected from an electrical change, a change in blood flow, a change in metabolic rate, or other changes) in the area to which the stimulation is applied. In the present invention, the intensity of the stimulation to be applied may be appropriately adjusted depending on the desired effect and the conditions (e.g., age, weight, degree of symptom) of the patient to be administered with the therapeutic agent.

As used herein, the term "stimulation of a nerve of the patient" means systemic or local application of stimulation to the patient in such a way as to cause any neural response. It should be noted that the term is not intended to exclude any mode in which the stimulation is also applied to any tissues or areas adjacent to the nerve. In the patient, the stimulation may be applied to only a single nerve area or to two or more nerve areas.

In the present invention, the stimulation may be applied at one or more time points selected from the time points before, during, or after the administration of the therapeutic agent of the present invention.

The stimulation applied to the patient may be any type capable of causing nerve stimulation. For easy enhancement of the therapeutic effect according to the present invention, the stimulation is preferably one or more selected from the group consisting of motor stimulation, sensory stimulation, electrical stimulation, magnetic stimulation, language stimulation, and higher brain function stimulation. One stimulation means may be used to apply one of these stimulations or to simultaneously apply two or more of these stimulations. For example, a volitional control electrical stimulation device may be used to simultaneously apply motor stimulation, sensory stimulation (somatosensory stimulation), and electrical stimulation to the patient. A robotic-assisted training device (e.g., Robot Suit HAL (trademark)) may be used in combination to simultaneously apply motor stimulation and sensory stimulation to the patient.

### [Motor Stimulation]

As used herein, the term "motor stimulation" means affected area-targeted motor stimulation. It should be noted, however, that "affected area-targeted motor stimulation" is not intended to exclude any motor stimulation associated with full-body workout (e.g., exercise with treadmill). Examples of motor stimulation include motor stimulation produced using neuromuscular facilitation technique (e.g., PNF method, Brunnstrom method, Bobath method) in combination to increase the quantity of stimulus to the target nerve pathway; motor stimulation produced by intensive repetitive movement, such as Kawahira method (repetitive facilitative exercise) or arm basis training; motor stimulation produced by forced exercise, such as constraint-induced movement therapy (CI therapy); and motor stimulation produced in combination with sensory stimulation, electrical stimulation, or magnetic stimulation. A robotic-assisted training device (e.g., Robot Suit HAL (trademark)) may also be used to simultaneously apply repetitive motor and sensory stimulations to the patient.

For example, a dysphagic patient preferably receives any type of motor stimulation in combination with swallowing training. A dysarthric patient preferably receives any type of motor stimulation in combination with articulation training.

Motor stimulation is stimulation of motor nerve pathways. Motor nerve pathways are pathways that transmit motor information from upper motor neurons (beginning in the cerebral cortex primary motor area or brainstem) to lower motor neurons. These pathways include lateral corticospinal tract, rubrospinal tract, reticulospinal tract, vestibulospinal tract, tectospinal tract, and corticobulbar tract. The motor nerve pathway also synapses with lower motor neurons, of which the axons extend as peripheral nerves and synapse with extrafusal muscle fibers to contract the target muscle and thus to cause movement. The primary motor area is modulated by premotor area, supplementary motor area, cingulate motor area, thalamus, primary somatosensory area, superior parietal lobule, and other areas, and the motor and sensory nerve pathways constantly modulate each other while they function.

### [Sensory Stimulation]

As used herein, the term "sensory stimulation" means stimulation of any of the senses related to nerve damage (e.g., visual sense, auditory sense, tactile sense). For ease of achieving the advantageous effect of the present invention, the sensory stimulation in the present invention is preferably selected from somatosensory stimulation, auditory stimulation, and visual stimulation.

As used herein, the term "somatosensory stimulation" collectively means cutaneous sensation, deep sensation, and visceral sensation. Specific examples of somatosensory stimulation include sensations produced in the skin, mucous membranes, joints, muscles, tendons, and other tissues (e.g., pain sensation, temperature sensation (cold to hot sensation), touch-pressure sensation).

Any suitable means may be used to apply somatosensory stimulation to the patient, such as tactile pressure, acupuncture or moxibustion, thermotherapy, weight band training, or vibration.

The tactile pressure may be applied by rehabilitation of the area with nerve damage (e.g., hand, foot), such as massage performed while the area with damage is visually checked.

The acupuncture or moxibustion may be performed by a method using needles or moxibustion (burning moxa).

The thermotherapy may be performed by a method using moxibustion (burning moxa), hot packs, or hydrotherapy.

The weight band training may be performed by a method using a weight band for light-load resistance exercise for rehabilitation.

The vibration may be performed by a method using a vibrator.

As used herein, the term "auditory stimulation" means stimulation provided by sound. The sound may be, but not limited to, human voice or other voice sound or any suitable music (e.g., a certain rhythmic rhythm).

The means for delivering auditory stimulation to the patient may be, but not limited to, rhythmic auditory stimulation (RAS).

As used herein, the term "visual stimulation" means stimulation provided by visual information. The visual information may be, but not limited to, any suitable information provided in the space (e.g., letters, pictures, images).

The means for delivering visual stimulation to the patient may be, but not limited to, functional training (e.g., visual search task, visual scanning training), activities of daily living (e.g., eating, clothing, toileting, grooming, bathing, reading, painting), or prismatic adaptation.

Sensory stimulation is usually stimulation of somatic sensations (sensations produced in the skin, mucous membranes, joints, muscles, tendons, or other tissues). Somatic sensations are broadly classified into four modalities (pain sensation, temperature sensation, touch-pressure sensation, and deep (proprioceptive) sensation), each of which is received through specialized sensory receptors, nerve fibers, and conduction pathways. Sensory neural pathways by which somatic sensations travel to the cerebral cortex have been identified, such as those shown below. Deep sensation and fine tough-pressure sensation travel through the posterior column-medial lemniscus system (receptors → first-order neurons (entering the spinal cord, ascending the ipsilateral posterior column, and terminating in the ipsilateral posterior column nuclei) → second-order neurons (crossing, ascending the contralateral medial lemniscus, and terminating in the contralateral thalamus VPL) → third-order neurons (reaching the contralateral cortical somatosensory area)). Pain and temperature sensation and coarse touch-pressure sensation travel through the spinal thalamic tract (receptors → first-order neurons (entering the spinal cord and terminating in the dorsal horn of the spinal cord) → second-order neurons (crossing, ascending the contralateral anterolateral funiculus, ascending the contralateral spinal thalamic tract, and terminating in the contralateral thalamus VPL) → third-order neurons (reaching the contralateral cortical somatosensory area)).

### [Electrical Stimulation]

As used herein, the term "electrical stimulation" means stimulation electrically delivered using electric current. For example, the electrical stimulation may be stimulation that excites neural circuits by applying electricity (e.g., low frequency, medium frequency, high frequency, interference waves) to the patient through electrodes attached to the affected area.

The means for delivering electrical stimulation to the patient may be, but not limited to, a method using an integrated volitional control electrical stimulator (e.g., IVES), a method conventionally known as current stimulation therapy (e.g., transcutaneous electrical nerve stimulation (TENS)), functional electrical stimulation (FES) technique, therapeutic electrical stimulation (TES) technique, transcranial direct current stimulation (tDCS), or deep brain stimulation (DBS) technique.

Electrical stimulation is usually performed by delivering stimulation, such as low frequency, medium frequency, high frequency, or interference waves, to paralyzed limbs through electrodes attached thereto to repeatedly excite neural circuits. The excitation of neural circuits by electrical stimulation can raise the pain threshold, as described by the gate-control theory, or lower the motor threshold to make it easier to move the paralyzed limb.

### [Magnetic Stimulation]

As used herein, the term "magnetic stimulation" means stimulation magnetically delivered using a static magnet or an electromagnet.

The means for delivering magnetic stimulation to the patient may be, but not limited to, a method conventionally known as magnetic stimulation (e.g., transcranial magnetic stimulation (TMS), transcranial direct current stimulation (tDCS), which uses small current for stimulation, or deep brain stimulation (DBS) therapy, which applies continuous electrical stimulation to the nervous system through electrodes inserted deep in the brain to therapeutically control its function). Typical examples of TMS include classical rTMS techniques, such as low-frequency rTMS (1 Hz or less), which acts in an inhibitory manner, and high-frequency rTMS (5 Hz or more), which acts in an excitatory manner. Alternatively, theta burst stimulation (TBS) may be used, which provides a burst stimulation of three continuous 50 Hz pulses, repeated at a frequency of 5 Hz. Intermittent TBS (iTBS) may be used, which includes performing theta burst stimulation (three continuous 50 Hz pulses at 5 Hz) for 2 seconds, followed by 8 seconds of rest, to increase excitability in the motor area. Continuous TBS (cTBS) may also be used, which includes continuously performing theta burst stimulation (three continuous 50 Hz pulses at 5 Hz) to decrease excitability in the motor area. In other words, TBS produces an inhibitory effect when performed continuously and produces an excitatory effect when performed intermittently. TBS is characterized in that it can be performed at a stimulation intensity lower than that for low- or high-frequency rTMS and has longer-lasting effects.

### [Language Stimulation]

As used herein, the term "language stimulation" means stimulation provided by language-mediated communication.

The means for delivering language stimulation to the patient may be, but not limited to, encouraging the patient to do an activity, such as reading, writing, drawing, listening, speaking, repetition, or calculation.

Language stimulation is usually stimulation of language-related brain areas (e.g., Broca's area, Wernicke's area, left angular gyrus (Broadman's area 39), left supramarginal gyrus (Broadman's area 40), cerebellum, thalamus, basal ganglia).

### [Higher Brain Function Stimulation]

As used herein, the term "higher brain function" is a general term for mental (psychological) functions including cognitive processes (e.g., perception, memory, learning, thinking, judgment) and emotions (feelings) for actions. As used herein, the term "higher brain function stimulation" is intended to include memory training, attention training, executive function training, and social behavior training.

The means for delivering higher brain function stimulation to the patient may be, but not limited to, encouraging the patient to undergo memory training, attention training, executive function training, social behavior training, or other training.

The higher brain function stimulation, when targeted for frontal lobe function, is usually stimulation of any stage in a pyramidal hierarchy, like awakening (bottom layer) → suppression and excitation → attention and concentration→ information processing → memory → executive function and logical thinking (see Comprehensive Rehabilitation May 2006 Issue published by Igaku-Shoin).

### [Other Stimulations]

As used herein, the term "stimulation" is intended to include not only the stimulations described above but also any stimulation that will produce a physiological change in the patient. For example, the stimulation may be full-body workout or the like in a case where it is applied during a period from before to 6 hours after the administration of the therapeutic agent.

### [Check and Evaluation of Stimulation]

In the present invention, whether and at what intensity the stimulation is applied to the patient may be identified by determining whether and at what level physiological changes occur in the patient.

Physiological changes that can be used as indicators regarding the stimulation include electrical changes, changes in blood flow, and changes in metabolic rate (e.g., metabolic rate of oxygen). Usually, the stronger the stimulation applied, the greater the amount of change in these physiological properties. For example, the stronger the stimulation applied, the more the blood flow in the brain.

Electrical changes may be determined by noninvasive brain function measurement, such as electroencephalography (EEG) or magnetoencephalography (MEG).

Changes in blood flow may be determined by noninvasive brain function measurement, such as functional magnetic resonance imaging (functional MRI) or single photon emission CT (SPECT).

Changes in cerebral blood flow and changes in oxygen metabolism may be determined by optical topography, positron emission tomography (PET), or other inspection techniques.

### [Timing of Stimulation]

The stimulation may be performed once or two or more times at any time point or points during the period from before to 6 hours after the administration of the therapeutic agent. It should be noted that this is not intended to exclude applying the stimulation at a time point more than 6 hours after the administration of the therapeutic agent.

Before the administration of the therapeutic agent, the stimulation may be applied at any appropriate timing determined according to the type of the stimulation, the conditions of the patient, or other factors.

The time taken for the stimulation to increase the blood flow to the affected area (e.g., the area of nerve damage) or the time for which the increase in blood flow is maintained may vary depending on the type of the stimulation. Therefore, the timing of the administration of the therapeutic agent is preferably adjusted to overlap when the affected area has a larger blood flow. Specifically, the time taken for the the application of stimulation to increase the blood flow to the affected area tends to be longer in a case where the stimulation is sensory or language stimulation than in a case where the stimulation is electrical or magnetic stimulation. Therefore, in a case where the stimulation is sensory or language stimulation, the period from the application of the stimulation to the administration of the therapeutic agent is preferably set relatively long (preferably within 6 hours before the administration, more preferably within 3 hours before the administration), or the period from the administration of the therapeutic agent to the application of the stimulation is preferably set relatively short (preferably within 3 hours after the administration, more preferably within 1 hour after the administration). In a case where the stimulation is electrical or magnetic stimulation, the period from the application of the stimulation to the administration of the therapeutic agent is preferably set relatively short (preferably within 3 hours before the administration, more preferably within 1 hour before the administration), or the period from the administration of the therapeutic agent to the application of the stimulation may be set relatively long (within 6 hours after the administration, preferably within 3 hours after the administration).

### [Area to Be Stimulated]

The stimulation may be preferentially applied to one or more selected from the group consisting of an area of nerve damage, an area near an area of nerve damage, and an area capable of compensating for the function of an area of nerve damage. This is not intended to exclude applying the stimulation to any area other than these areas.

As used herein, the expression "preferentially applied (to the specified area)" means that when the stimulation started, the stimulation is initially applied to at least one selected from an area of nerve damage, an area near an area of nerve damage, or an area capable of compensating for the function of an area of nerve damage.

As used herein, the term "an area of nerve damage" means an area itself having nerve damage (e.g., atrophy, nerve blockage, transection, rupture, loss, brain damage, spinal cord injury). Usually, the area of nerve damage causes neurological disease.

As used herein, the term "an area near an area of nerve damage" means an area that exists near an area of nerve damage, not being the area of nerve damage itself (e.g., an area surrounding the periphery of an area of nerve damage or an area adjacent to an area of nerve damage).

As used herein, the term "an area capable of compensating for the function of an area of nerve damage" means an area that acts to compensate for the function of an area of nerve damage (e.g., in the case of damage to the primary motor area, the right parietal lobe near the affected area, the unaffected primary motor area, the premotor area, or the supplementary motor area).

Examples of the area to be stimulated in the patient include head (e.g., brain), face, eyes, ears, mouth, upper limbs, lower limbs, trunk, articulatory organs, and swallowing organs.

Hereinafter, examples of the area to which the stimulation may be applied and examples of the method for applying the stimulation will be described for each type of stimulation.

### [Examples of How to Apply Motor Stimulation]

In the case of nerve damage to the right primary motor area (hand control area), the "area of nerve damage" is the right primary motor area (hand control area). In the case of nerve damage to the right primary motor area (hand control area), the "area near the area of nerve damage" may be an area from an unaffected part of the right primary motor area (other than the hand control area) to the premotor area, the supplementary motor area, or the right primary sensory area. In the case of nerve damage to the right primary motor area (hand control area), the "area capable of compensating for the function of the area of nerve damage" may be the right parietal lobe, an area from the left primary motor area to the premotor area, or the supplementary motor area.

In the case of nerve damage to the right primary motor area (hand control area), an attempt to move the paralyzed left-hand finger will preferentially provide stimulation to the area of nerve damage. In the case of nerve damage to the right primary motor area (hand control area), an attempt to move an area adjacent to the paralyzed left-hand finger (e.g., an unaffected left-hand finger, the left wrist) will preferentially provide stimulation to an area near the area of nerve damage. In the case of nerve damage to the right primary motor area (hand control area), an attempt to use the paralyzed left-hand finger to do various tasks or an attempt to simply move the paralyzed left-hand finger will preferentially provide stimulation to an area capable of compensating for the function of the area of nerve damage.

### [Examples of How to Apply Sensory Stimulation]

In the case of nerve damage to the right primary sensory area (third-order neurons), the "area of nerve damage" is the right primary sensory area. In the case of nerve damage to the right primary sensory area (third-order neurons), the "area near the area of nerve damage" may be an area from an unaffected part of the right primary sensory area to the right primary motor area. In the case of nerve damage to the right primary sensory area (third-order neurons), the "area capable of compensating for the function of the area of nerve damage" may be the left primary sensory area, the secondary somatosensory area communicating with the primary sensory area, the parietal association area, the motor area, or the visual area.

In the case of nerve damage to the right primary sensory area (third-order neurons), sensory stimulation may be applied to the affected area with impaired sensation so that the stimulation can be preferentially applied to the area of nerve damage. In the case of nerve damage to the right primary sensory area (third-order neurons), sensory stimulation may be applied to an area adjacent to the area with impaired sensation so that the stimulation can be preferentially applied to the area near the area of nerve damage. In the case of nerve damage to the right primary sensory area (third-order neurons), sensory stimulation may be applied to the area with impaired sensation or an area near the impaired sensation area in the patient making visual confirmation or simultaneously receiving the same level of sensory stimulation at the unaffected area so that the stimulation can be preferentially applied to the area capable of compensating for the function of the area of nerve damage.

### [Examples of How to Apply Electrical Stimulation]

In the case of nerve damage to the right second finger, the "area of nerve damage" is the right second finger. In the case of nerve damage to the right second finger, the "area near the area of nerve damage" may be the right first, third, fourth, or fifth finger with no nerve damage. In the case of nerve damage to the right second finger, the "area capable of compensating for the function of the area of nerve damage" may be the right wrist, forearm, upper arm, or shoulder.

In the case of nerve damage to the right second finger, electrical stimulation may be applied to the right second finger (the area of nerve damage) so that the stimulation can be preferentially applied to the area of nerve damage. In the case of nerve damage to the right second finger, electrical stimulation may be applied to the right first, third, fourth, or fifth finger, which is near the right second finger with nerve damage, so that the stimulation can be preferentially applied to the area near the area of nerve damage. In the case of nerve damage to the right second finger, electrical stimulation may be applied to the right wrist, forearm, upper arm, or shoulder so that the stimulation can be preferentially applied to the area capable of compensating for the function of the area of nerve damage.

### [Examples of How to Apply Magnetic Stimulation]

In the case of nerve damage to the right primary motor area (hand control area), the "area of nerve damage" is the right primary motor area (hand control area). In the case of nerve damage to the right primary motor area (hand control area), the "area near the area of nerve damage" may be an area from an unaffected part of the right primary motor area (other than the hand control area) to the premotor area, the supplementary motor area, or the right primary sensory area. In the case of nerve damage to the right primary motor area (hand control area), the "area capable of compensating for the function of the area of nerve damage" may be the right parietal lobe, an area from the left primary motor area to the premotor area, or the supplementary motor area.

In the case of nerve damage to the right primary motor area (hand control area), excitatory magnetic stimulation (e.g., high-frequency rTMS (5 Hz or more) or intermittent TBS) may be applied to the right primary motor area (hand control area) so that the stimulation can be preferentially applied to the area of nerve damage. In the case of nerve damage to the right primary motor area (hand control area), excitatory magnetic stimulation may be applied to an area near the area of nerve damage, from part of the right primary motor area (other than hand control area) to the premotor area, the supplementary motor area, or the right primary sensory area so that the stimulation can be preferentially applied to the area near the area of nerve damage. In the case of nerve damage to the right primary motor area (hand control area), excitatory magnetic stimulation may be applied to the right parietal lobe so that the stimulation can be preferentially applied to the area capable of compensating for the function of the area of nerve damage. Moreover, inhibitory magnetic stimulation (e.g., low-frequency rTMS (1 Hz or less) or continuous TBS) may be applied to the left primary motor area to reduce the inhibition (interhemispheric inhibition) of the activity from the left to the right cerebral hemisphere, so that the area of nerve damage (the right primary motor area (hand control area)), the near area (the area from part of the right primary motor area (other than hand control area) to the premotor area, the supplementary motor area, or the right primary sensory area), or the compensation area (the right parietal lobe) can be relieved from the inhibition and that blood flow and excitability can be increased in these areas.

### [Examples of How to Apply Language Stimulation]

In the case of nerve damage to the Wernicke's area, the "area of nerve damage" is the Wernicke's area. In the case of nerve damage to the Wernicke's area, the "area near the area of nerve damage" may be an unaffected part of the Wernicke's area, the Broca's area, or language circuits including conduction pathways (arcuate fasciculus) connecting the two areas. In the case of nerve damage to the Wernicke's area, the "area capable of compensating for the function of the area of nerve damage" may be the left angular gyrus (Broadman's area 39), left supramarginal gyrus (Broadman's area 40), cerebellum, thalamus, or basal ganglia.

In the case of nerve damage to the Wernicke's area, sensory language stimulation may be applied so that the stimulation can be preferentially applied to the area of nerve damage. In the case of nerve damage to the Wernicke's area, repetition practice or motor language stimulation may be performed so that the stimulation can be preferentially applied to the area near the area of nerve damage. In the case of nerve damage to the Wernicke's area, a task such as phonological training, vocabulary training, grammar training, reading training, or calculation training may be provided so that the stimulation can be preferentially applied to the area capable of compensating for the function of the area of nerve damage.

### [Examples of How to Apply Higher Brain Function Stimulation]

In the case of nerve damage causing executive function disorder, the "area of nerve damage" is a brain area that controls the executive function. In the case of nerve damage causing executive function disorder, the "area near the area of nerve damage" may be a brain area that controls memory or information processing at a layer lower than that for executive function. In the case of nerve damage causing executive function disorder, the "area capable of compensating for the function of the area of nerve damage" may be a brain area that controls attention, concentration, inhibition, activation, arousal, etc. at a layer lower than that for memory or information processing.

In the case of nerve damage causing executive function disorder, executive function training may be performed to preferentially apply the stimulation to the area of nerve damage. In the case of nerve damage causing executive function disorder, training of memory or information processing at a layer lower than that for executive function may be performed to preferentially apply the stimulation to the area near the area of nerve damage. In the case of nerve damage causing executive function disorder, training for attention, concentration, inhibition, activation, or arousal at a layer lower than that for memory or information processing may be performed to preferentially apply the stimulation to the area capable of compensating for the function of the area of nerve damage.

### EXAMPLES

Hereinafter, the present invention will be described in more detail with reference to examples, which are not intended to limit the present invention.

### <Mesenchymal Stem Cells>

A liquid agent containing mesenchymal stem cells at a concentration of 1 × 10⁶ cells/mL was prepared according to the Example 3 method disclosed in Japanese Patent No. 4061487.

### <Treatment of Patients>

The mesenchymal stem cell preparation was intravenously administered to patients having different nerve damage symptoms as shown below. In combination with the administration, stimulation (one selected from motor stimulation, sensory stimulation, electrical stimulation, magnetic stimulation, language stimulation, and higher brain function stimulation) was applied to the patients.

### (Intravenous Administration of Mesenchymal Stem Cells)

Each of the patients was administered with one dose of 100 mL of the liquid agent by drip infusion over 1 hour.

### (Stimulation)

Stimulation was applied to each patient as described in detail below.

Hereinafter, the expression "stimulation was applied 1 hour before the administration of mesenchymal stem cells" means that the time point at which the stimulation was started was 1 hour before the time point at which the administration of mesenchymal stem cells was started. The expression "stimulation was applied during the administration of mesenchymal stem cells" means that the time point at which the stimulation was started was during the administration of mesenchymal stem cells (at any time point during the 1-hour cell administration period). The expression "stimulation was applied n hours after the administration of mesenchymal stem cells" means that the time point at which the stimulation was started was n hours after the time point at which the administration of mesenchymal stem cells was started.

### [Motor Stimulation]

A 48-year-old male patient with cerebral hemorrhage was treated by repetitive facilitative exercise therapy for motor stimulation. In this case, the stimulation was applied to an area of nerve damage (at left fingers and from left foot to lower leg), an area near the area of nerve damage (from left wrist to forearm and from left knee joint to thigh), and an area capable of compensating for the function of the area of nerve damage (from left elbow joint to shoulder joint and at left hip joint). The motor stimulation was applied 6 hours and 1 hour before the administration of mesenchymal stem cells, during the administration of mesenchymal stem cells, and 3, 6, and 12 hours after the administration of mesenchymal stem cells. The time per application of the stimulation was 30 minutes for each of the areas. In addition, a 50-year-old male patient with the same condition did full-body workout (30 minutes per treadmill exercise session) and received the administration of mesenchymal stem cells as shown above.

### [Sensory Stimulation]

A 48-year-old male patient with cerebral hemorrhage received sensory stimulation to his upper and lower limbs with paresthesia. The sensory stimulation was ice bag cooling stimulation to the paralyzed limbs. The patient received the stimulation while receiving auditory stimulation (speaking to the patient) and being instructed to visually confirm the position of the upper and lower limbs. In this case, the stimulation was applied to an area of nerve damage (at left fingers and from left foot to lower leg), an area near the area of nerve damage (from left hand joint to forearm and from left knee joint to thigh), and an area capable of compensating for the function of the area of nerve damage (from left elbow joint to shoulder joint and at left hip joint). The sensory stimulation was applied 6 hours and 1 hour before the administration of mesenchymal stem cells, during the administration of mesenchymal stem cells, and 3, 6, and 12 hours after the administration of mesenchymal stem cells. The time per application of the stimulation was 30 minutes for each of the areas. The sensory stimulation was applied to the affected upper and lower limbs. In addition, a 50-year-old male patient with the same condition received the sensory stimulation (30 minutes per application) to his unaffected upper and lower limbs and received the administration of mesenchymal stem cells as shown above.

### [Electrical Stimulation]

A 48-year-old male patient with cerebral hemorrhage received electrical stimulation to his paralyzed upper and lower limbs. The electrical stimulation was low-frequency stimulation, which was applied to the patient through electrodes attached to his paralyzed hand and foot. In this case, the stimulation was applied to an area of nerve damage (at left fingers and from left foot to lower leg), an area near the area of nerve damage (from left wrist joint to forearm and from left knee joint to thigh), and an area capable of compensating for the function of the area of nerve damage (from left elbow joint to shoulder joint and at left hip joint). The electrical stimulation was applied 6 hours and 1 hour before the administration of mesenchymal stem cells, during the administration of mesenchymal stem cells, and 3, 6, and 12 hours after the administration of mesenchymal stem cells. The time per application of the stimulation was 20 minutes for each of the areas. The electrical stimulation was applied to the affected upper and lower limbs. In addition, a 50-year-old male patient with the same condition received the electrical stimulation (20 minutes per application) to his unaffected upper and lower limbs and received the administration of mesenchymal stem cells as shown above.

### [Magnetic Stimulation]

A 48-year-old male patient with cerebral hemorrhage received transcranial magnetic stimulation to his head from a TMS system (manufactured by CR Technology). Specifically, the stimulation was intermittent TBS (iTBS) (1 burst of 3 stimuli at 50 Hz) applied at an intensity of 80% exercise threshold at 5 Hz (time intervals of 200 ms). The procedure of stimulation for 2 seconds and rest for 8 seconds was repeated to apply 2,000 pulses in total. In this case, the stimulation was applied to an area of nerve damage (a hand control area of the affected primary motor area), an area near the area of nerve damage (other than the hand control area of the affected primary motor area), and an area capable of compensating for the function of the area of nerve damage (right parietal lobe). The magnetic stimulation was applied 6 hours and 1 hour before the administration of mesenchymal stem cells, during the administration of mesenchymal stem cells, and 3, 6, and 12 hours after the administration of mesenchymal stem cells. The time per application of the stimulation was approximately 11 minutes (2,000 pulses) for each of the areas. The magnetic stimulation was applied to the affected head area. In addition, a 50-year-old male patient with the same condition received placebo magnetic stimulation (sound only) (approximately 11 minutes per application of 2,000 pulses) to the affected head area and received the administration of mesenchymal stem cells as shown above. During the placebo magnetic stimulation to the affected head area, no magnetic stimulation was actually applied to the patient.

### [Language Stimulation]

An 81-year-old female patient with cerebral hemorrhage and aphasia received language stimulation. The language stimulation was applied using a communication task (reading, writing, listening, speaking, repetition, and calculation). The language stimulation was applied 6 hours and 1 hour before the administration of mesenchymal stem cells, during the administration of mesenchymal stem cells, and 3, 6, and 12 hours after the administration of mesenchymal stem cells. The time for which the stimulation was applied was 60 minutes.

### [Higher Brain Function Stimulation]

An 81-year-old female patient with brain hemorrhage and higher brain dysfunction (attention deficit) received higher brain function stimulation. The higher brain function stimulation was applied by attention process training. The higher brain function stimulation was applied 6 hours and 1 hour before the administration of mesenchymal stem cells, during the administration of mesenchymal stem cells, and 3, 6, and 12 hours after the administration of mesenchymal stem cells. The time for which the stimulation was applied was 60 minutes.

### [Reference Test]

For reference, an 81-year-old female patient with cerebral hemorrhage and aphasia was allowed to passively listen to the radio for 1 hour or passively watch television for 1 hour. This stimulation was applied 6 hours and 1 hour before the administration of mesenchymal stem cells, during the administration of mesenchymal stem cells, and 3, 6, and 12 hours after the administration of mesenchymal stem cells.

<Efficacy of the Nerve Damage Therapy>

After each of the treatments was performed on the patients, each of the symptoms was evaluated using the indicator shown below for evaluation of the therapeutic effect. The results of the evaluation were classified according to the criteria shown below. The results are shown in Table 1.

### (Evaluation)

Paralysis and Sensory Impairment: Stroke Impairment Assessment (SIAS) and Fugl-Meyer Assessment (FMA)
Brain infarction: National Institutes of Health Stroke Scale (NIHSS)
Aphasia: Standard Language Test of Aphasia (SLTA)
Higher brain function: Mini-Mental State Examination (MMSE) [0116]

### (Evaluation Criteria for Motor Stimulation)

⊙: A marked therapeutic effect was observed as compared to that resulting from the full-body workout (12 hours after the cell administration).
○: A certain therapeutic effect was observed as compared to that resulting from the full-body workout (12 hours after the cell administration).
Δ: A slight therapeutic effect was observed as compared to that resulting from the full-body workout (12 hours after the cell administration).

### (Evaluation Criteria for Sensory Stimulation and Electrical Stimulation)

⊙: A marked therapeutic effect was observed as compared to that resulting from the stimulation of the unaffected area (12 hours after the cell administration).
○: A certain therapeutic effect was observed as compared to that resulting from the stimulation of the unaffected area (12 hours after the cell administration).
Δ: A slight therapeutic effect was observed as compared to that resulting from the stimulation of the unaffected area (12 hours after the cell administration).

### (Evaluation Criteria for Magnetic Stimulation)

⊙: A marked therapeutic effect was observed as compared to that resulting from the placebo magnetic stimulation to the affected area (12 hours after the cell administration).
○: A certain therapeutic effect was observed as compared to that resulting from the placebo magnetic stimulation to the affected area (12 hours after the cell administration).
Δ: A slight therapeutic effect was observed as compared to that resulting from the placebo magnetic stimulation to the affected area (12 hours after the cell administration).

### (Evaluation Criteria for Language Stimulation, Higher Brain Function Stimulation, and Reference Test)

⊙: A significant therapeutic effect was observed as compared to that observed before the cell administration and stimulation.
○: A certain therapeutic effect was observed as compared to that observed before the cell administration and stimulation.
Δ: A slight therapeutic effect was observed as compared to that observed before the cell administration and stimulation.
X: No therapeutic effect was observed as compared to that observed before the cell administration and stimulation.

**[Table 1]**

| | Motor stimulation | | | Full-body workout |
|---|---|---|---|---|
| | Area of nerve damage | Area near area of nerve damage | Area capable of compensating for the function of area of nerve damage | |
| 6 hours before cell administration | Δ | Δ | Δ | - |
| 1 hour before cell administration | ○ | ○ | ○ | Δ |
| During cell administration | ⊙ | ⊙ | ⊙ | Δ |
| 3 hours after cell administration | ⊙ | ⊙ | ⊙ | Δ |
| 6 hours after cell administration | ⊙ | ⊙ | ⊙ | Δ |
| 12 hours after cell administration | ○ | ○ | ○ | - (Comparison reference) |

**[Table 2]**

| | Sensory stimulation to affected area | | | Sensory stimulation to unaffected area |
|---|---|---|---|---|
| | Area of nerve damage | Area near area of nerve damage | Area capable of compensating for the function of area of nerve damage | |
| 6 hours before cell administration | Δ | Δ | Δ | - |
| 1 hour before cell administration | ○ | ○ | ○ | - |
| During cell administration | ⊙ | ⊙ | ⊙ | - |
| 3 hours after cell administration | ⊙ | ⊙ | ⊙ | - |
| 6 hours after cell administration | ⊙ | ⊙ | ⊙ | - |
| 12 hours after cell administration | Δ | Δ | Δ | - (Comparison reference) |

**[Table 3]**

| | Electrical stimulation to affected area | | | Electrical stimulation to unaffected area |
|---|---|---|---|---|
| | Area of nerve damage | Area near area of nerve damage | Area capable of compensating for the function of area of nerve damage | |
| 6 hours before cell administration | Δ | Δ | Δ | - |
| 1 hour before cell administration | ○ | ○ | ○ | - |
| During cell administration | ⊙ | ⊙ | ⊙ | - |
| 3 hours after cell administration | ⊙ | ⊙ | ⊙ | - |
| 6 hours after cell administration | ⊙ | ⊙ | ⊙ | - |
| 12 hours after cell administration | Δ | Δ | Δ | - (Comparison reference) |

**[Table 4]**

| | Magnetic stimulation to affected area | | | Placebo magnetic stimulation to affected area |
|---|---|---|---|---|
| | Area of nerve damage | Area near area of nerve damage | Area capable of compensating for the function of area of nerve damage | |
| 6 hours before cell administration | Δ | Δ | Δ | - |
| 1 hour before cell administration | ○ | ○ | ○ | - |
| During cell administration | ⊙ | ⊙ | ⊙ | - |
| 3 hours after cell administration | ⊙ | ⊙ | ⊙ | - |
| 6 hours after cell administration | ⊙ | ⊙ | ⊙ | - |
| 12 hours after cell administration | Δ | Δ | Δ | - (Comparison reference) |

**[Table 5]**

| | Language stimulation | Higher brain function stimulation | Reference test (Radio) | Reference test (Television) |
|---|---|---|---|---|
| 6 hours before cell administration | Δ | Δ | Δ | Δ |
| 1 hour before cell administration | ○ | ○ | Δ | Δ |
| During cell administration | ⊙ | ⊙ | Δ | Δ |
| 3 hours after cell administration | ⊙ | ⊙ | Δ | Δ |
| 6 hours after cell administration | ⊙ | ⊙ | Δ | Δ |
| 12 hours after cell administration | Δ | Δ | X | X |

As shown above, a significant amelioration of the nerve damage symptom was observed when the therapeutic agent was used in a manner satisfying the requirements according to the present invention.

## Claims

1. A therapeutic agent for treatment of nerve damage,
the therapeutic agent being an intravenously administered formulation comprising mesenchymal stem cells and/or cells capable of differentiating into mesenchymal stem cells,
the therapeutic agent being to be administered to a patient in combination with stimulation of a nerve of the patient,
the stimulation being to be performed during a period from before to 6 hours after the administration of the therapeutic agent.

2. A therapeutic agent for treatment of nerve damage,
the therapeutic agent being an intravenously administered formulation comprising mesenchymal stem cells and/or cells capable of differentiating into mesenchymal stem cells,
the therapeutic agent being to be administered to a patient in combination with stimulation of a nerve of the patient,
the stimulation being to be preferentially applied to at least one selected from the group consisting of an area of nerve damage, an area near an area of nerve damage, and an area capable of compensating for a function of an area of nerve damage.

3. The therapeutic agent according to claim 1 or 2, wherein the stimulation is at least one selected from the group consisting of motor stimulation, sensory stimulation, electrical stimulation, magnetic stimulation, language stimulation, and higher brain function stimulation.
